# DEMANDE DE BREVET EUROPEEN

(11) **EP 1 213 059 A1**
(43) Date de publication de la demande: **12.06.2002**
(21) Numéro de dépôt: 01403079.5
(22) Date de dépôt: 30.11.2001
(51) Int. Cl.: B09B 3/00, C12M 1/107

(54) **Procédé de traitement de farines contaminées**

(30) Priorité: 07.12.2000 FR 0015872
(71) Demandeur: Tschocke, Christian, 82800 Bruniquel (FR)
(72) Inventeur: Tschocke, Christian, 82800 Bruniquel (FR)
(74) Mandataire: Fruchard, Guy

(57) **Abrégé**

Procédé de traitement de farines contaminées comprenant les étapes de provoquer une fermentation bactériologique génératrice de gaz combustible, recueillir le gaz combustible obtenu, clarifier la liqueur résultant de la fermentation pour séparer des boues et une phase aqueuse, extraire de la phase aqueuse les éléments contaminants sous forme d'une saumure concentrée, et incinérer la saumure et les boues à une température suffisante pour détruire les éléments contaminants.

## Description

La présente invention concerne un procédé de traitement de farines contaminées, en particulier des farines animales contaminées par des protéines mutantes.

On sait que l'interdiction récente de l'utilisation des farines animales dans l'alimentation des bovidés pose non seulement le problème de la destruction du stock existant de farines animales mais également celui de la destruction des carcasses animales résultant de la consommation quotidienne de viande.

On a envisagé d'effectuer cette incinération dans des cimenteries afin de profiter des températures élevées obtenues dans les fours de cimenteries et provoquer ainsi la destruction des protéines mutantes qui sont détruites à partir d'une température de 360°C.

Toutefois, l'incinération dans les fours des cimenteries pose différents problèmes. Tout d'abord cette incinération pose un problème de transport, les farines animales n'étant généralement pas stockées à proximité d'une cimenterie. Elle pose ensuite un problème de coût, les fours des cimenteries étant alimentés par un combustible externe. Et elle pose surtout un problème de risque de contamination lors des manipulations, les cimenteries n'étant généralement pas prévues pour recevoir des produits dangereux.

Selon l'invention on propose un procédé de traitement de farines contaminées comprenant les étapes de provoquer une fermentation bactériologique génératrice de gaz combustible, recueillir le gaz combustible obtenu, clarifier la liqueur résultant de la fermentation pour séparer des boues et une phase aqueuse, extraire de la phase aqueuse les éléments contaminants sous forme d'une saumure concentrée, et incinérer la saumure et les boues à une température suffisante pour détruire les éléments contaminants.

Ainsi, l'énergie nécessaire pour l'incinération des éléments contaminants est extraite des farines à traiter de sorte que le bilan énergétique est équilibré voire même favorable, la fermentation bactériologique produisant généralement une quantité de gaz combustible supérieure à la quantité nécessaire pour l'incinération des éléments contaminants. Il est alors possible d'envisager l'investissement d'une chaîne de traitement spécifique a proximité des lieux de stockage des farines animales ou des lieux des productions des carcasses animales en prenant des dispositions nécessaires pour éviter une contamination lors du traitement de sorte que l'on supprime tout à la fois le risque de contamination et le coût du transport.

Selon une version avantageuse du procédé suivant l'invention, celui-ci comporte en outre une étape préalable d'hydrolyse des farines par action enzymatique. On diminue ainsi le poids moléculaire moyen, ce qui favorise l'action des bactéries lors de la fermentation bactériologique et améliore le rendement énergétique du traitement.

Selon un autre aspect avantageux de l'invention la saumure concentrée est obtenue par une évaporation sous vide suivie d'une filtration par osmose inverse. On réduit ainsi de façon notable la quantité d'eau associée aux éléments contaminants avant leur incinération de sorte que le bilan énergétique se trouve encore amélioré et d'autre part on recueille à la sortie du filtre osmotique une eau débarrassée de tout élément contaminant qui peut être rejetée sans risquer une pollution de la nappe phréatique, ou de préférence réutilisée pour provoquer la fermentation bactériologique d'un nouveau lot de farines contaminées introduit dans l'unité de traitement.

D'autres caractéristiques et avantages de l'invention apparaîtront à la lecture de la description qui suit d'un mode de mise en oeuvre particulier non limitatif du procédé selon l'invention.

Le procédé selon l'invention est de préférence mis en oeuvre en continu. Au démarrage d'un cycle, un lot de farines contaminées est disposé dans une cuve contenant de l'eau et des enzymes, cette cuve étant maintenue à une température appropriée connue en soi pour que l'action enzymatique provoque une hydrolyse des molécules à très haut poids moléculaire, de sorte qu'en fin de réaction la bouillie obtenue est d'un poids moléculaire homogène faible favorable à une métabolisation de bactéries. La bouillie est alors transférée dans un fermenteur où elle est soumise à un traitement bactériologique également connu en soi destiné à favoriser la production de méthane en évitant la production de métabolites intermédiaires qui peuvent être toxiques. La fermentation bactériologique ainsi réalisée est donc productrice à titre principal de méthane et de dioxyde de carbone. Le dioxyde de carbone est éliminé par un lavage du gaz et le méthane est stocké en vue de son utilisation ultérieure ainsi qu'il sera vu ci-après.

En fin de l'étape de fermentation bactériologique, la liqueur résultant de la fermentation est clarifiée, par exemple par passage dans des ouvrages de sédimentation, de façon à séparer les boues d'une part et une phase aqueuse d'autre part. Les boues sont stockées en vue de leur incinération ainsi qu'il sera indiqué ci-après et la phase aqueuse est traitée tout d'abord par évaporation sous vide suivie d'une filtration par osmose inverse. L'évaporation sous vide permet de recueillir une première partie d'eau purifiée qui est renvoyée vers la première cuve en tête de process, et une solution concentrée qui est soumise à la filtration osmotique. Dans la filtration osmotique, les protéines mutantes sont trop grosses pour passer à travers la membrane de sorte que la filtration osmotique permet de recueillir une seconde partie d'eau purifiée qui est renvoyée vers la première cuve et une saumure concentrée contenant les protéines mutantes.

Cette saumure ainsi que les boues recueillies lors de l'étape de clarification sont envoyées dans un four alimenté par le méthane recueilli dans l'étape de fermentation bactériologique. La température du four est de préférence maintenue à 525°C afin d'assurer non seulement une évaporation de l'eau résiduelle, mais également de porter les protéines mutantes à une température au moins égale à 360°C à laquelle elles sont détruites.

Bien entendu, l'invention n'est pas limitée au mode de mise en oeuvre décrit et est susceptible de variantes de réalisation sans sortir du cadre de l'invention tel que défini par les revendications.

En particulier, bien que le procédé selon l'invention ait été décrit en relation avec le traitement de farines animales comportant des molécules à haut poids moléculaire et pour lesquelles il est donc préférable d'effectuer une première étape d'action enzymatique, le procédé selon l'invention peut être utilisé avec d'autres farines contaminées, par exemple des farines végétales qui, si elles contiennent une faible proportion de molécules à haut poids moléculaire, pourront être traitées en procédant directement à l'étape de fermentation bactériologique.

De même, si une évaporation sous vide s'avère suffisante pour obtenir une saumure concentrée pouvant être directement incinérée, on pourra prévoir de supprimer l'étape de filtration osmotique. On notera toutefois que la suppression de cette étape de filtration osmotique ne permettra pas de recueillir la seconde partie d'eau purifiée et il sera donc nécessaire de faire un apport d'eau plus important au départ d'un cycle.

Bien que dans le mode de mise en oeuvre préféré on ait prévu une incinération à une température préférée de 525°C, on pourra prévoir une température plus basse à condition de laisser les produits à incinérer pendant un temps suffisant dans le four pour s'assurer que les protéines mutantes sont portées à une température de 360°C. Cette température pourra bien entendu être adaptée en fonction des éléments contaminants à détruire.

Bien qu'il ait été prévu ci-dessus de réutiliser le méthane produit pour l'incinération des boues et de la saumure, on peut si on le souhaite récupérer le méthane pour une autre utilisation et utiliser un autre combustible pour l'incinération, le bilan énergétique global étant de toutes façons plus favorable en utilisant le procédé de l'invention.

## Revendications

1. Procédé de traitement de farines contaminées **caractérisé en ce qu'**il comprend les étapes de provoquer une fermentation bactériologique génératrice de gaz combustible, recueillir le gaz combustible obtenu, clarifier la liqueur résultant de la fermentation pour séparer des boues et une phase aqueuse, extraire de la phase aqueuse les éléments contaminants sous forme d'une saumure concentrée, et incinérer la saumure et les boues à une température suffisante pour détruire les éléments contaminants.

2. Procédé selon la revendication 1, **caractérisé en ce qu'**il comporte une étape préalable d'hydrolyse des farines par action enzymatique.

3. Procédé selon la revendication 1 ou la revendication 2, **caractérisé en ce que** la saumure concentrée est obtenue par évaporation sous vide suivie d'une filtration par osmose inverse.

4. Procédé selon l'une quelconque des revendications précédentes, **caractérisé en ce que** l'incinération est réalisée en utilisant le gaz combustible produit lors de l'étape de fermentation bactériologique.

5. Procédé selon l'une quelconque des revendications précédentes, **caractérisé en ce que** l'incinération est effectuée à au moins 400°C, de préférence au moins à 500°C.
